(19) Europäisches Patentamt
European Patent Office
Office européen des brevets

(11) EP 1 579 843 A2

(12) EUROPEAN PATENT APPLICATION

(43) Date of publication:
**28.09.2005 Bulletin 2005/39**

(51) Int Cl.$^7$: **A61K 7/06**

(21) Application number: **05006538.2**

(22) Date of filing: **24.03.2005**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **25.03.2004 JP 2004089741**

(71) Applicant: **KAO CORPORATION**
**Tokyo 103-8210 (JP)**

(72) Inventors:
- **Fukuhara, Kazuhisa**
  **Tokyo 131-8501 (JP)**
- **Ueyama, Kenichi**
  **Tokyo 131-8501 (JP)**

(74) Representative: **HOFFMANN EITLE**
**Patent- und Rechtsanwälte**
**Arabellastrasse 4**
**81925 München (DE)**

# (54) Hair cosmetic composition

(57) Provided is a leave-on hair cosmetic composition, which contains the following components (A) and (B):

(A) a citric acid or salt thereof; and
(B) at least one or more of an organic solvent selected from the group consisting of aromatic alcohols, N-alkylpyrrolidones, alkylene carbonates, polypropylene glycols, lactones and cyclic ketones, wherein the organic solvent has a ClogP of from -2 to 3;

and wherein the leave-on hair cosmetic composition has a pH of from 2 to 5 at 25°C when diluted to 20 times the weight with water and has a buffering capacity of 0.001 gram equivalent/L or greater but less than 0.2 gram equivalent/L. Provided also is a hair quality improving method which comprises treating the hair with the hair cosmetic composition.

The hair cosmetic composition of the present invention can provide benefits such as luster, manageability and good feel of the hair to hair which is apt to be dry and moistureless owing to damage by coloring, permanent weaving, or repetition of excessive blow drying.



Printed by Jouve, 75001 PARIS (FR)

**Description**

FIELD OF THE INVENTION

The present invention relates to a leave-on hair cosmetic composition containing a citric acid for improving strength/body, manageability, setting power and feel of the hair.

BACKGROUND OF THE INVENTION

In recent years, it has been said that owing to the influence of chemical treatment such as hair coloring or physical treatment by blow drying, cuticles on the hair surface peel off or the hair becomes porous by the efflux of lipids from the inside of the hair and as a result, the hair inevitably becomes excessively dry, resistant to finger combing, difficult to style and manage, and lusterless.

Commercially available leave-on hair cosmetic compositions which have been popularly used for providing the hair with manageability and protecting the hair from excessive drying are emulsion type products such as hair cream having wax, higher alcohol and surfactant, and gels having a film forming polymer (set polymer). Such hair cosmetic compositions can temporarily overcome the problems such as poor manageability and excessive dryness by causing an oil or fat or a polymer to adhere to the hair surface, but cannot fundamentally improve the hair luster or manageability.

Some hair cosmetic compositions for improving the hair quality are known. Of these, compositions using a specific organic acid and organic solvent which employ a technology intended to improve the hair quality by acting on the inside of the hair are known (refer to, for example, JP-A-1995-112921, JP-A-1994-172131, JP-A-1997-301831 and JP-A-1994-298625). These compositions promote manageability of the hair by softening the hair which is stiff and therefore, hard to handle.

SUMMARY OF THE INVENTION

In the present invention, there is thus provided a leave-on hair cosmetic composition, which contains the following components (A) and (B):

(A) a citric acid or salt thereof, and
(B) at least one or more of an organic solvent selected from the group consisting of aromatic alcohols, N-alkylpyrrolidones, alkylene carbonates, polypropylene glycols, lactones and cyclic ketones, wherein the organic solvent has a ClogP of from -2 to 3; and

wherein the leave-on hair cosmetic composition has a pH of from 2 to 5 at 25°C when diluted to 20 times the weight with water and has a buffering capacity of 0.001 gram equivalent/L or greater but less than 0.2 gram equivalent/L.

In another aspect of the invention, there is also provided a hair quality improving method, which comprises treating the hair with the above-described composition.

DETAILED DESCRIPTION OF THE INVENTION

The present invention relates to a leave-on hair cosmetic composition capable of for example essentially improving the hair quality, improving luster, manageability and setting power of the hair, and providing excellent feel of the hair. In the present invention, the term "hair quality improvement" means improvement of strength/body, manageability and feel of the hair.

The present inventors have found that by giving a certain buffering performance to a hair cosmetic composition containing citric acid and a penetration-promoting organic solvent, the resulting composition may stably maintain a performance resistant against environmental changes upon using (for example, when applied to a wet hair), promotes the penetration of citric acid and organic solvent into the hair, and gives further improved strength/body, manageability and feel to the hair.

As Component (A), the salt of citric acid is, for example, a salt with an alkali metal, alkaline earth metal, ammonia or organic amine compound. The content of citric acid or salt thereof as Component (A) in the hair cosmetic composition of the present invention is preferably from 0.01 to 30 wt.%, more preferably from 0.1 to 20 wt.%, even more preferably from 0.5 to 10 wt.% in consideration of internal hair-quality improving effect (pore repairing effects, and the like), set retention improving effect and manageability improving effect.

The organic solvent to be used as Component (B) is selected from the group consisting of aromatic alcohols, N-alkylpyrrolidones, alkylene carbonates, polypropylene glycols, lactones and cyclic ketones. Specific examples include the following (b1) to (b5), respectively.

(b1) Aromatic alcohols represented by the formula (1) :

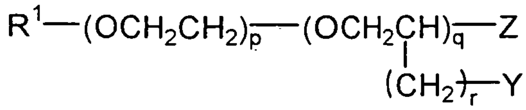

(1)

wherein, $R^1$ represents a group $R^2$-Ph-$R^3$- ($R^2$: a hydrogen atom, a methyl group or a methoxy group, $R^3$: a bond or a saturated or unsaturated divalent $C_{1-3}$ hydrocarbon group, Ph: paraphenylene group), Y and Z each represents a hydrogen atom or a hydroxy group, and p, q and r each stands for an integer of from 0 to 5, with the proviso that when p=q=0, Z does not represent a hydrogen atom and $R^1$ does not represent a group $R^2$-Ph-.

(b2) N-alkylpyrrolidones having a nitrogen atom to which a $C_{1-18}$ alkyl group has been bonded.

(b3) $C_{2-4}$ Alkylene carbonates.

(b4) Polypropylene glycols having a number average molecular weight of from 100 to 1000.

(b5) Lactones or cyclic ketones represented by any one of the formulas (2), (3) and (4):

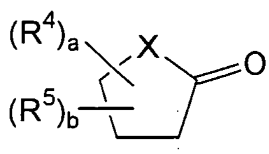

(2)

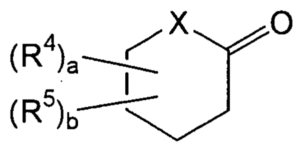

(3)

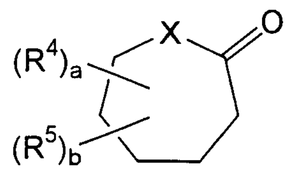

(4)

wherein, X represents a methylene group or an oxygen atom, $R^4$ and $R^5$ respectively represent substituents which are different from each other, and a and b each stands for 0 or 1.

Examples of (b1) include benzyl alcohol, cinnamyl alcohol, phenethyl alcohol, p-anisyl alcohol, p-methylbenzyl alcohol, phenoxyethanol, and 2-benzyloxyethanol; those of (b2) include N-methylpyrrolidone, N-octylpyrrolidone and N-laurylpyrrolidone; and those of (b3) include ethylene carbonate and propylene carbonate. As (b4), polypropylene glycols having a number average molecular weight of from 100 to 500 are preferred, with a number average molecular weight of from 300 to 500 being more preferred.

In (b5), $R^4$ and $R^5$ in the formulas (2) to (4) are each preferably a linear, branched or cyclic alkyl group, hydroxy group, sulfonic acid group, phosphoric acid group, carboxy group, phenyl group, sulfoalkyl group, phosphoric acid alkyl group and carboxyalkyl group. When (b5) is a lactone, γ-lactone and δ-lactone are preferred. Described specifically, as $R^4$ or $R^5$, lactones having a linear or branched $C_{1-6}$ alkyl group (methyl, ethyl, propyl, isopropyl, butyl or the like) replaced with the methylene adjacent to the hetero oxygen atom are preferred. In order to enhance the water solubility of the lactone or cyclic ketone represented by (2) to (4), $R^4$ or $R^5$ preferably represents an acid group such as sulfonic acid group, phosphoric acid group or carboxy group, or an alkyl group substituted therewith. Specific examples of the lactone include γ-butyrolactone, γ-caprolactone, γ-valerolactone, δ-valerolactone, δ-caprolactone and δ-heptanolactone. Of these, γ-lactone are preferred, with γ-butyrolactone and γ-caprolactone being more preferred. Examples of the cyclic ketone include cyclopentanone, cyclohexanone, cycloheptanone and 4-methylcycloheptanone.

Component (B) is preferably a liquid at 25°C and has a ClogP of from -2 to 3, preferably from -1 to 2 in view of penetration promotion. The term "ClogP" as used herein means a measure indicating the distribution.of a substance between an octanol phase and an aqueous phase. It is a calculated value of an octanol-water distribution coefficient (logP) as defined by the below-described equation and its examples are described in Chemical Reviews, 71(6), 1971.

$$\log P = \log ([\text{Substance}]_{octanol} / [\text{Substance}]_{water})$$

wherein, $[\text{Substance}]_{octanol}$ means a mole concentration of a substance in a 1-octanol phase, while $[\text{Substance}]_{water}$ means a mole concentration of the substance in an aqueous phase.

The ClogP of each of the main compounds usable as Component (B) is as follows: benzyl alcohol (1.1), 2-benzyloxyethanol (1.2), 2-phenylethanol (1.2), 1-phenoxy-2-propanol (1.1), polypropylene glycol 400 (0.9), propylene

carbonate (-0.41), and γ-butyrolactone (-0.64).

Preferred examples of Component (B) include benzyl alcohol, 2-benzyloxyethanol, propylene carbonate and polypropylene glycol (preferably, number average molecular weight of 400).

As Component (B), two or more compounds may be used in combination. Their content in the hair cosmetic composition of the invention is preferably from 0.1 to 40 wt.%, more preferably from 0.5 to 10 wt.%, even more preferably from 1 to 5 wt.% in view of its feeling upon use, hair luster and promotion of hair quality improving effects (improvement of elasticity, improvement of moisture resistance, and the like).

A weight ratio (A):(B) of citric acid or salt thereof as Component (A) to the organic solvent as Component (B) preferably ranges from 10:1 to 1:7, more preferably from 4:1 to 1:3 in order to effectively develop internal hair-quality improving (pore repairing) effect, set retention improving effect and manageability improving effect.

The hair cosmetic composition of the invention may further contain ethanol. Ethanol contributes to the solubilization or stable dispersion of Component (B). The content of ethanol in the hair cosmetic composition of the invention is preferably from 0.01 to 50 wt.%, more preferably from 1 to 20 wt.%. A weight ratio of ethanol to Component (B) preferably ranges from 40:1 to 2:1, more preferably from 20:1 to 3:1 from the viewpoint of penetration promotion of Components (A) and (B) into the hair.

The hair cosmetic composition of the invention may further contain a set polymer in view of improvement of hair styling property, regulation of viscosity, stability, improvement of adhesion upon application to the hair, improvement of feel of the hair and early expression of hair quality improving effects. Examples of such a polymer include polyvinylpyrrolidone polymer compounds such as polyvinylpyrrolidone, vinylpyrrolidone/vinyl acetate copolymer, vinylpyrrolidone/vinyl acetate/vinyl propionate tertiary copolymer, vinylpyrrolidone/alkylaminoacrylate (quaternary chloride) copolymer, vinylpyrrolidone/acrylate/(meth)acrylic acid copolymer, and vinylpyrrolidone/alkylaminoacrylate/vinylcaprolactam copolymer; acidic vinyl ether polymer compounds such as methyl vinyl ether/maleic anhydride alkyl half ester copolymer; acidic polyvinyl acetate polymer compounds such as vinyl acetate/crotonic acid copolymer, vinyl acetate/crotonic acid/vinyl neodecanoate copolymer and vinyl acetate/crotonic acid /vinyl propionate copolymer; acidic acrylic polymer compounds such as (meth)acrylic acid/(meth)acrylate copolymer, and acrylic acid/alkyl acrylate/alkylacrylamide copolymer; amphoteric acrylic polymer compounds such as N-methacryloylethyl-N,N-dimethylammonium-α-N-methylcarboxybetaine/butyl methacrylate copolymer, and hydroxypropyl acrylate/butylaminoethyl methacrylate/acrylic octylamide copolymer; basic acrylic polymer compounds such as acrylamide/acrylate quaternary copolymer; cellulose derivatives such as cationic cellulose derivative; and chitin/chitosan derivatives such as hydroxypropyl chitosan, carboxymethyl chitin, and carboxymethyl chitosan.

These set polymers may be used either singly or in combination of two or more. Their content in the hair cosmetic composition of the invention is preferably from 0.1 to 10 wt.%, more preferably from 0.5 to 5 wt.%.

In the hair cosmetic composition of the invention, a conditioning component selected from silicones and oily substances may be incorporated in order to improve conditioning effects further. Examples of the silicones include dimethylpolysiloxanes, methylphenylpolysiloxane, cyclic silicones, polyether-modified silicones, amino-modified silicones, carboxy-modified silicones, fatty acid-modified silicones, alcohol-modified silicones, aliphatic alcohol-modified silicones, epoxy-modified silicones, fluorine-modified silicones, and alkyl-modified silicones. Of these, dimethylpolysiloxanes, polyether-modified silicones and amino-modified silicones are preferred.

As the dimethylpolysiloxane, those having a viscosity of from 5 $mm^2/s$ to 10 million $mm^2/s$ can be used depending on the desired feel of the hair, wherein those having a viscosity of 10 million $mm^2/s$ are often supplied in the form of an emulsion. Of these, those having a viscosity falling within a range of from 5000 $mm^2/s$ to 10 million $mm^2/s$, are preferred, with those having a viscosity falling within a range of from 50000 $mm^2/s$ to 10 million $mm^2/s$ being more preferred because they can impart the hair with good lubricity.

The term "polyether-modified silicones" is a generic name of polyoxyethylene/methylpolysiloxane copolymers and poly(oxyethylene/oxypropylene)methylpolysiloxane copolymers. They can impart the hair with smoothness. The polyether-modified silicones having various HLBs are known. Examples of the commercially available products thereof include "Silicone KF351A", "Silicone KF353A", "Silicone KF6008", "Silicone KF6016", "Silicone KF6011", and "Silicone KF6012" (each, trade name; product of Shin-etsu Chemical), and "SH3771C", "SH3773C", and "SH3775C" (each, trade name; product of Dow Corning Toray Silicone).

As the amino-modified silicones, amodimethicone oil or an emulsion thereof is preferred, because they can impart the hair with moistness. Their commercially available products are amodimethicone emulsion "SM8704C" (trade name; product of Dow Corning Toray Silicone) and "KT-1989" and "XF-42-B1989" (each, trade name; product of GE Toshiba Silicones).

In the invention, various silicones can be used either singly or in combination of two or more, depending on the performance desired. The content of the silicones in the hair cosmetic composition of the invention is preferably from 0.05 to 20 wt.%, more preferably from 0.1 to 10 wt.%, even more preferably from 0.5 to 5 wt.% in consideration of smoothness to facilitate finger combing and stickiness-free feel.

The oily substance is preferably added to improve the hair manageability after drying. Examples thereof include hydrocarbons such as squalene, squalane, liquid isoparaffin, light liquid isoparaffin, heavy liquid isoparaffin, α-olefin oligomer, liquid paraffin and cycloparaffin; glycerides such as castor oil, cacao oil, mink oil, avocado oil and olive oil; waxes such as bees wax, sperm wax, lanolin, microcrystalline wax, ceresin wax and carnauba wax; higher alcohols such as cetyl alcohol, oleyl alcohol, stearyl alcohol, isostearyl alcohol and 2-octyldodecanol; esters such as octyldodecyl myristate, hexyl laurate, cetyl lactate, propylene glycol monostearate, oleyl oleate, hexadecyl 2-ethylhexanoate, isononyl isononanoate and tridecyl isononanoate; higher fatty acids such as capric cid, lauric acid, myristic acid, palmitic acid, stearic acid, behenic acid, oleic acid, coconut oil fatty acid, isostearic acid and isopalmitic acid; and other oils such as isostearyl glyceryl ether and polyoxypropylene butyl ether. Of these, hydrocarbons, such as branched hydrocarbons including squalene, squalane, liquid isoparaffin, light liquid isoparaffin, heavy liquid isoparaffin, α-olefin oligomer are preferred.

These oily substances may be used either singly or in combination of two or more. Their content in the hair cosmetic composition of the invention is preferably from 0.05 to 20 wt.%, more preferably from 0.1 to 10 wt.%, even more preferably from 0.5 to 5 wt.% in view of good manageability and stickiness-free feel.

In the hair cosmetic composition of the invention, a surfactant may be incorporated from the viewpoints of stability of the system including the solubilization or dispersion of the solvent, and improvement in the feel of the hair. As the surfactant, any one of cationic surfactant, nonionic surfactant, amphoteric surfactant and anionic surfactant can be used.

Examples of the cationic surfactant include quaternary ammonium salts represented by the following formula (5):

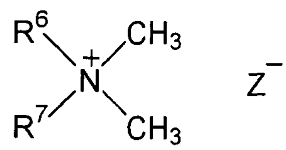
(5)

wherein $R^6$ and $R^7$ each independently represents a hydrogen atom, a $C_{1-28}$ alkyl group or a benzyl group, with the proviso that they do not simultaneously represent a hydrogen atom, a benzyl group or a $C_{1-3}$ lower alkyl group, and Z- represents an anion.

Either one of $R^6$ and $R^7$ preferably represents an alkyl group having from 16 to 24 carbon atoms, more preferably 22 carbon atoms, preferably a linear alkyl group, while the other one represents a lower $C_{1-3}$ alkyl group, preferably a methyl group. Examples of the anion $Z^-$ include halide ions such as chloride ions and bromide ions, and organic anions such as ethyl sulfate ions and methyl carbonate ions. Of these, halide ions are preferred, with chloride ions being preferred.

As the cationic surfactant, mono(long chain alkyl) quaternary ammonium salts are preferred. Specific examples include cetyltrimethylammonium chloride, stearyltrimethylammonium chloride, arachyltrimethylammonium chloride and behenyltrimethylammonium chloride. Of these, stearyltrimethylammonium chloride and behenyltrimethylammonium chloride are preferred.

Examples of the nonionic surfactant include polyoxyalkylene alkyl ethers, polyoxyalkylene alkenyl ethers, higher fatty acid sucrose esters, polyglycerin fatty acid esters, higher fatty acid mono- or diethanolamides, polyoxyethylene hydrogenated castor oils, polyoxyethylene sorbitan fatty acid esters, polyoxyethylene sorbitol fatty acid esters, alkyl saccharide surfactants, alkylamine oxides, and alkyl amideamine oxides. Of these, polyoxyalkylene alkyl ethers and polyoxyethylene hydrogenated castor oils are preferred, with polyoxyethylene alkyl ethers being more preferred.

As the amphoteric surfactant, imidazoline, carbobetaine, amidobetaine, sulfobetaine, hydroxysulfobetaine, and amidosulfobetaine can be used.

Examples of the anionic surfactant include alkylbenzene sulfonates, alkyl or alkenyl ether sulfates, alkyl or alkenyl sulfates, olefin sulfonates, alkane sulfonates, saturated or unsaturated fatty acid salts, alkyl or alkenyl ether carboxylates, α-sulfone fatty acid salts, N-acylamino acid surfactants, mono- or di-phosphate surfactants and sulfosuccinates. Examples of the counterion to the anionic residue of the above-described surfactants include alkali metal ions such as sodium ion and potassium ion; alkaline earth metal ions such as calcium ion and magnesium ion, ammonium ions, alkanolamines having 1 to 3 alkanol groups with 2 or 3 carbon atoms (such as monoethanolamine, diethanolamine, triethanolamine and triisopropanolamine). Examples of the counterion to the cationic residue include halide ions such as chloride ions, bromide ions and iodide ions, methosulfate ions and saccharinate ions.

Of these, cationic surfactants are preferred in view of feel of the hair.

These surfactants may be used either singly or in combination of two or more. The content of the surfactant (s) in the hair cosmetic composition of the invention is preferably from 0.01 to 10 wt.%, more preferably from 0.05 to 3 wt.% in view of stability of the system including solubilization of the solvent and emulsification of an oily substance.

The hair cosmetic composition of the invention may further contain a polyhydric alcohol. The polyhydric alcohol contributes to solubilization and stable dispersion of Component (B). In addition, the improvement of luster and enhancement of the hair quality improving effect are accelerated by the synergistic action between the polyhydric alcohol and Component (B). Examples of the polyhydric alcohol include ethylene glycol, glycerin, sorbitol, propylene glycol, 1,3-butyleneglycol and dipropylene glycol. Of these, glycerin is preferred. These polyhydric alcohols may be used either singly or in combination of two or more. Their content in the hair cosmetic composition of the invention is preferably from 0.1 to 10 wt.%, more preferably from 0.5 to 5 wt.%.

The hair cosmetic composition of the invention may further contain, as needed, components employed for ordinary hair cosmetic compositions depending on their purpose of use. Examples of such components include antidandruffs, vitamin preparations, bactericides, antiinflammatories, antiseptics, chelating agents, humectants coloring agents such as dyes and pigments, viscosity regulators such as hydroxyethyl cellulose, methyl cellulose, polyethylene glycol and clay mineral, plant extracts, pearling agents, perfumes, colorants, ultraviolet absorbers, antioxidants, and the other components as described in the ENCYCLOPEDIA OF SHAMPOO INGREDIENTS (MICELLE PRESS).

The hair cosmetic composition of the invention is adjusted to have a pH of from 2 to 5 (at 25°C when diluted to 20 times the weight with water), preferably from 2.5 to 4, more preferably from 3 to 4 in order to develop the performances of the composition fully, for example, to improve the strength/body and luster of the hair and to give flexibility, manageability and pliability to the hair. Use of sodium hydroxide or potassium hydroxide is preferred to adjust the pH to fall within the above-described range.

Moreover, for controlling a change in pH upon application to the hair, promoting the penetration of the organic acid and organic solvent to the hair, causing the hair quality improving effects to appear promptly and improving the feeling, the hair cosmetic composition of the invention has a buffering capacity, as a 5 wt.% aqueous solution, of 0.001 gram equivalent/L or greater but less than 0.2 gram equivalent/L, preferably from 0.003 gram equivalent/L or greater but less than 0.1 gram equivalent/L, more preferably from 0.005 gram equivalent/L or greater but less than 0.05 gram equivalent/L. The term "buffering capacity" as used herein means a value determined from the below-described equation with, as a scale, the concentration of a base necessary for raising the pH of a 5 wt.% aqueous solution by 1 from the initial pH at 25°C.

$$\text{Buffering capacity} = |dC_B/dpH|$$

wherein, $C_B$ represents the ion concentration (gram equivalent/L) of a base.

Such a buffering capacity can be given to the hair cosmetic composition by adding thereto a pH buffering agent. As the pH buffering agent, organic acids or inorganic acids, or salts thereof having a buffering action within a pH range of from 2 to 5 can be employed. Examples of the organic acid include, in addition to citric acid as Component (A), malonic acid, succinic acid, glutaric acid, adipic acid, maleic acid, fumaric acid, phthalic acid, oxalic acid, malic acid, tartaric acid, lactic acid, revulinic acid, butyric acid, valeric acid and mandelic acid; those of the inorganic acid include phosphoric acid, sulfuric acid and nitric acid; and those of the salt of these acids include alkali metal salts such as sodium salt and potassium salt, ammonium salts, and alkanolamine salts such as triethanolamine salt. No particular limitation is imposed on the amount of the pH buffering agent and it varies depending on the kind of the compound used to give a buffering capacity. When sodium citrate is mainly used as a compound for giving a buffering capacity, it is added in an amount of about 1 wt.% or greater.

The form of the hair cosmetic composition of the invention may be selected from liquid, gel, paste, cream and wax as needed, but when in the form of a solution using a solvent, water or a lower alcohol is preferred as the solvent, with water being more preferred.

The hair cosmetic composition of the invention is preferably used as a hair styling agent or hair conditioning agent. It can be provided, for example, as a pump spray, aerosol spray, pump foam, aerosol foam, gel or lotion.

By heating after application of the hair cosmetic composition of the invention to the hair, penetration of Components (A) and (B) into the hair can be accelerated. For heating, a drier, heater or hair curling iron can be used. The heating temperature is preferably 60°C or greater, more preferably 70°C or greater.

## Examples

The present invention will hereinafter be described in further detail by examples. It should however be borne in mind that the present invention is not limited to or by them.

The pH in the below-described examples and comparative example are those at 25°C when diluted to 20 times the weight with water.

Example 1

Hair cosmetic compositions as shown in Table 2 were prepared and their "setting property", "strength/body improving effect", "manageability", "feel of the hair" and "luster" were evaluated. The results are shown in Table 2.

(Evaluation method)

Evaluation of "setting property"

1) Hair bundle to be evaluated

A hair bundle of 10 cm in length, 1.5 cm in width and 1 g in weight was made using the hair of a Japanese female whose hair was not subjected to chemical treatment such as permanent waving or hair coloring. The hair bundle was bleached (by "Ravenus Color Appeal Inazuma Bleach"; product of Kao) twice and the resulting hair bundle was provided for the evaluation of setting property.

2) Treatment of the hair bundle

Pre-shampoo evaluation (treated 7 times)

The hair bundle to be evaluated was subjected to shampooing (with "Ravenus Designing Shampoo", product of Kao), towel drying, uniform application of 0.1 g of the invention product or comparative product (which will hereinafter be called "treating agent"), and drying for 10 minutes with hot air of 70°C. This treatment was repeated six times in total. After shampooing, towel drying and application (the seventh application) of the treating agent similarly, the hair bundle was wound around a rod having a diameter of 4 cm and dried for 10 minutes with hot air of 70°C.

Post-shampoo evaluation

The internal hair-quality improving effects were studied by evaluating the set retention property after the treating agent was washed away from the hair surface. After completion of the pre-shampoo evaluation, each hair bundle was shampooed and towel dried. Without application of the treating agent to the hair, the hair bundle was wound around a rod having a diameter of 4 cm and dried for 10 minutes with hot air of 70°C.

3) Procedures and criteria of evaluation

The curled bundle was removed from the rod and a comb (ring comb) was run through the bundle 20 times to disentangle it. It was suspended in a thermo-hygrostat (25°C and 98%RH) to determine the set retention power. The set retention power was determined in the following manner. The length of the hair bundle thus suspended (distance from the bundled position to the end of the hair) was measured. Supposing that the length of the hair bundle just after suspension was a length at set retention of 100% and the initial length (10 cm) of the hair bundle before curling was a length at a set retention ratio of 0%, a relative length (%) of the hair bundle after 30 minutes, that is, a set retention ratio was determined in accordance with the following equation:

$$\text{Set retention (\%)} = \frac{\text{(initial length of the hair bundle) -(length of the hair bundle after 30 minutes)}}{\text{(initial length of the hair bundle) - (length of the hair bundle just after curling)}} \times 100$$

Evaluation of "strength/body improving effect", "manageability", "feel of the hair (smoothness, moistness, softness, stiffness, stickiness)" and "luster"

1) Hair bundle to be evaluated

A hair bundle of 25 cm in length and 6 g in weight was made using the hair of a Japanese female whose hair was not subjected to chemical treatment such as permanent waving or hair coloring. The hair bundle was bleached (by "Ravenus Color Appeal Inazuma Bleach"; trade name; product of Kao) twice and the resulting hair bundle was provided for the evaluation.

2) Treatment of the hair bundle

Pre-shampoo evaluation

The hair bundle to be evaluated was subjected to shampooing (with "Ravenus Designing Shampoo", trade name; product of Kao), towel drying, uniform application of 0.6 g of the treating agent, and drying for 10 minutes with hot air of 70°C while running a ring comb through the hair bundle. This treatment was repeated seven times in total.

Post-shampoo evaluation

In order to study the internal hair-quality improving effect, the hair bundle after completion of the pre-shampoo evaluation was shampooed and towel-dried, and then dried for 10 minutes with hot air of 70°C while running a ring comb through the hair bundle.

3) Evaluation criteria

Organoleptic evaluation by a panel of 5 experts was performed in accordance with the criteria shown in Table 1, and an average of the scores is shown in Table 2.

Table 1

| (Strength/body improving effects) | (Manageability) |
|---|---|
| 5: Obvious improvement in strength/body<br>4: Improvement in strength/body<br>3: Some improvement in strength/body<br>2: Only slight improvement in strength/body<br>1: No improvement in strength/body | 5: Excellent manageability<br>4: Some manageability<br>3: Cannot be said either way<br>2: A little inferior in manageability<br>1: Lack of manageability |
| (Feel of the hair: smoothness) | (Feel of the hair: moist feel) |
| 5: Very Smooth<br>4: Smooth<br>3: Cannot be said either way<br>2: Slightly smooth<br>1: Not smooth | 5: Very moist<br>4: Moist<br>3: Cannot be said either way<br>2: Slightly moist<br>1: Not moist |
| (Feel of the hair: softness) | (Feel of the hair: stiffness) |
| 5: Very soft<br>4: Soft<br>3: Cannot be said either way<br>2: Slightly soft<br>1: Not soft | 5: Not stiff<br>4: Slightly stiff<br>3: Cannot be said either way<br>2: Stiff<br>1: Very stiff |
| (Feel of the hair: stickiness) | (Luster) |
| 5: Not sticky<br>4: Slightly sticky<br>3: Cannot be said either way<br>2: Sticky<br>1: Very sticky | 5: Marked improvement in luster<br>4: Improvement in luster<br>3: Cannot be said either way<br>2: No improvement in luster<br>1: Loss of luster |

Table 2

| | | Example product | | Comparative product |
|---|---|---|---|---|
| | | 1 | 2 | 1 |
| Components mixed (wt.%) | Citric acid | 6.0 | 5.0 | 0.01 |
| | Malic acid | - | 1.5 | - |
| | 2-Benzyloxyethanol | 2.5 | 2.5 | 2.5 |
| | Ethanol | 10.0 | 10.0 | 10.0 |
| | Water | Balance | Balance | Balance |
| | Sodium hydroxide (pH buffer) | 1.18 | 1.30 | - |
| pH (25°C, when diluted to 20 times the weight with water) | | 3.71 | 3.70 | 3.35 |
| Buffering capacity | | 0.012 g/L | 0.014 g/L | 0.0005 g/L |
| Pre-shampoo evaluation | Set retention (%) | 82 | 78 | 36 |
| | Strength/body improving effects | 4.0 | 3.6 | 1.8 |
| | Manageability | 3.8 | 4.4 | 2.0 |
| | Smoothness | 3.6 | 4.0 | 1.6 |
| | Moist feel | 3.8 | 3.8 | 2.2 |
| | Softness | 3.6 | 4.2 | 3.0 |
| | Stiffness | 3.4 | 3.8 | 4.0 |
| | Stickiness | 4.2 | 4.0 | 4.2 |
| | Luster | 3.8 | 4.2 | 1.8 |
| Post-shampoo evaluation | Set retention (%) | 69 | 66 | 33 |
| | Strength/body improving effect | 3.8 | 3.6 | 1.8 |
| | Manageability | 3.2 | 3.8 | 2.0 |

It has been confirmed based on the above-described results that unlike hair cosmetic compositions which are available by the conventional technology which has not overcome the problems such as stiffness and stickiness, the composition according to the invention can attain good set retention, strength/body improving effects, manageability and feel of the hair improving effects. It has also been confirmed that the above-described effects last even after the removal of the components applied to the surface of the hair; and in addition, the improvement in hair quality such as elimination of pores inside of the hair can be observed.

Example 2: (Pump spray)

| | (wt.%) |
|---|---|
| Citric acid | 6.0 |
| Stearyltrimethylammonium chloride | 0.25 |
| Glycerin | 1.0 |
| 2-Benzyloxyethanol | 2.5 |
| Ethanol | 4.5 |
| Perfume | 0.02 |
| Water | Balance |
| Citric acid | 1.1 |

(continued)

| | (wt.%) |
|---|---|
| Sodium hydroxide (pH regulator) | Amount to adjust pH to 3.7 |
| [buffering capacity = 0.013 gram equivalent/L] | |

Example 3: (Pump mist)

| | (wt.%) |
|---|---|
| Citric acid | 4.5 |
| Malic acid | 1.5 |
| 2-Benzyloxyethanol | 2.5 |
| Polyvinylpyrrolidone | 3.0 |
| Ethanol | 10.0 |
| Perfume | 0.05 |
| Water | Balance |
| Sodium hydroxide (pH regulator) | Amount to adjust pH to 3.7 |
| [buffering capacity = 0.012 gram equivalent/L] | |

Example 4: (Hair gel)

| | (wt.%) |
|---|---|
| Citric acid | 3.5 |
| Succinic acid | 1.5 |
| Glycerin | 2.0 |
| 2-Benzyloxyethanol | 2.5 |
| Hydroxyethyl cellulose | 2.0 |
| Ethanol | 10.0 |
| Perfume | 0.05 |
| Water | Balance |
| Potassium hydroxide (pH regulator) | Amount to adjust pH to 3.7 |
| [buffering capacity = 0.007 gram equivalent/L] | |

Example 5: (Hair lotion)

| | (wt.%) |
|---|---|
| Citric acid | 5.0 |
| Lactic acid | 1.0 |
| Glycerin | 1.0 |
| 2-Benzyloxyethanol | 2.5 |
| Ethanol | 10.0 |
| Perfume | 0.02 |
| Water | Balance |
| Sodium hydroxide (pH regulator) | Amount to adjust pH to 3.7 |
| [buffering capacity = 0.012 gram equivalent/L] | |

Example 6: (Hair lotion)

| | (wt.%) |
|---|---|
| Citric acid | 3.0 |
| Lactic acid | 1.5 |
| Tartaric acid | 1.0 |
| Stearyltrimethylammonium chloride | 0.1 |
| Benzyl alcohol | 2.0 |
| Polyethylene glycol 400 | 0.45 |
| Ethanol | 4.5 |
| Water | Balance |
| Potassium hydroxide (pH regulator) | Amount to adjust pH to 3.7 |
| [buffering capacity = 0.010 gram equivalent/L] | |

Example 7 : (Pump foam)

| | (wt.%) |
|---|---|
| Citric acid | 2.5 |
| Succinic acid | 2.5 |
| Polyoxyethylene lauryl ether (16E.O.) | 1.0 |
| Stearyltrimethylammonium chloride | 0.1 |
| Glycerin | 1.0 |
| 2-Benzyloxyethanol | 2.0 |
| Ethanol | 4.0 |
| Perfume | 0.02 |
| Water | Balance |
| Sodium hydroxide (pH regulator) | Amount to adjust pH to 3.7 |
| [buffering capacity = 0.007 gram equivalent/L] | |

**Claims**

**1.** A leave-on hair cosmetic compositioncomprising the following components (A) and (B):

(A) a citric acid or salt thereof, and
(B) at least one or more of an organic solvent selected from the group consisting of aromatic alcohols, N-alkylpyrrolidones, alkylene carbonates, polypropylene glycols, lactones and cyclic ketones; wherein the organic solvent has a ClogP of from -2 to 3; and

wherein the leave-on hair cosmetic composition has a pH of from 2 to 5 at 25°C when diluted to 20 times the weight with water and has a buffering capacity of 0.001 gram equivalent/L or greater but less than 0.2 gram equivalent/L.

**2.** The hair cosmetic composition of Claim 1, further comprising a set polymer.

**3.** The hair cosmetic composition of Claim 1 or 2, further comprising at least one or more conditioning components selected from silicones and oily substances.

**4.** The hair cosmetic composition of any one of Claims 1 to 3, further comprising a cationic surfactant.

**5.** A hair quality improving methodcomprising the step of treating the hair with a hair cosmetic composition as claimed in any one of Claims 1 to 4.